(19) Europäisches Patentamt
European Patent Office
Office européen des brevets

(11) **EP 3 858 232 A1**

(12) **EUROPEAN PATENT APPLICATION**

(43) Date of publication:
**04.08.2021 Bulletin 2021/31**

(51) Int Cl.:
**A61B 5/0428** [(2006.01)]   **A61B 5/00** [(2006.01)]

(21) Application number: **20154351.9**

(22) Date of filing: **29.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **MEFTAH, Mohammed**
**5656 AE Eindhoven (NL)**

• **OUZOUNOV, Sotir Filipov**
**5656 AE Eindhoven (NL)**
• **ZHANG, Yijing**
**5656 AE Eindhoven (NL)**
• **HARPE, Pieter**
**5656 AE Eindhoven (NL)**

(74) Representative: **Philips Intellectual Property & Standards**
**High Tech Campus 5**
**5656 AE Eindhoven (NL)**

(54) **SENSING SYSTEM AND METHOD FOR ELECTROPHYSIOLOGICAL SENSING BY CAPACITIVE COUPLING WITH ESTIMATION OF THE ELECTRODE TO SKIN COUPLING**

(57) A sensing system and method uses a sense electrode arrangement for coupling to a surface of a body such that the sense electrode arrangement and the body (and the spacing between them) define a coupling capacitance. First and second sensing circuits have different transfer functions and generate first and second outputs. These outputs are processed to determine the coupling capacitance. The electrophysiological signal being monitored is also acquired by one or both of the sensing circuits. In this way, the quality of the electrode coupling can be determined in a simple and passive manner.

FIG. 2

**Description**

FIELD OF THE INVENTION

This invention relates to the sensing of electrophysiological signals based on sensing electrodes which are capacitively coupled to the surface of a body being monitored. The invention relates in particular to the estimation of the electrode to skin coupling in systems using such capacitive sensing electrodes.

BACKGROUND OF THE INVENTION

Measurements of electrophysiological signals such as ECG, EMG, and EEG signals, are required in professional health monitoring and diagnoses, and more and more in personal health products as well.

Traditionally these signals are measured with electrodes that are attached to the skin via a galvanic contact, often using electrolytic gel. Disadvantages of this way of measurement are mainly that it requires a long preparation time, causes skin irritation during prolonged usage, restricts the patient from free moving and provides less comfort for the patient, i.e. the patient is aware of being monitored.

Dry electrodes overcome some of the disadvantages as they do not need gel. However, this electrode type also requires a galvanic contact to the skin. Furthermore, after applying these electrodes, it takes time before the electrode-skin interface reaches a stable equilibrium, for example it typically takes 5-10 minutes before a baseline drift and a noise level have settled completely before a reliable measurement can be performed. In applications based on spot-checks this would be too long and is thus not acceptable.

A lot of research is currently being devoted to contactless (capacitive) measurement of electrophysiological signals in order to overcome the above-mentioned disadvantages. In this technique, a capacitor is effectively formed in which the human skin acts as one of the capacitor plates and the electrode of the sensor acts as the other capacitor plate. When using capacitive sensing, no galvanic contact to the skin is needed (i.e. the sensing is contactless).

Capacitive electrophysiological sensors can be integrated into a wide range of everyday objects, such as office chairs, clothing, cars and beds. The incorporation of sensors in clothing for example enables a long-term monitoring in an unobtrusive way.

Although there is no galvanic contact in capacitive electrophysiological measurements, just as for other types of electrophysiological measurement techniques, it is important that electrodes are coupled sufficiently to the body in order to ensure reliable monitoring. This means that the distance between the electrodes and the skin should be within a certain predefined range (typically less than 3mm for an electrode area of 1 cm$^2$), i.e. the capacitive coupling should be large enough (typically more than 1pF).

In order to guarantee a reliable measurement, it is desirable for the quality of the coupling to be communicated to the signal acquisition system. Thus, it is desirable for the measurement system to keep track of the electrode to skin coupling.

Known techniques for tracking the quality of the coupling are based on the injection of an electrical current of known frequency (e.g. 1 kHz) through the electrode to skin interface into the human body. By determining the amplitude of the corresponding output signal as picked up by the capacitive sensor, an estimate can be made of the electrode to skin coupling. A disadvantage of this method is that safety issues might play a role related to the injection of currents into the body, e.g. risks to the fetus in case of fetal monitoring applications. Even if the design is safe, it is complex due to the additional requirements that have to be addressed.

Despite the very attractive advantages of capacitive electrophysiological sensors, the issue of motion artefacts has remained one of the main unsolved issues which have hindered their reliable use in real-life applications. The movements of the user induce a change in the electrode to skin coupling, resulting in motion artefacts. The issue becomes more severe when static charges are trapped at the electrode to skin interface or in the case of tribo-electric discharges.

There is therefore a need for an improved system and method which can monitor the quality of the capacitive coupling of an electrode to the body of a subject.

SUMMARY OF THE INVENTION

The invention is defined by the claims.

According to examples in accordance with an aspect of the invention, there is provided a sensing system for sensing an electrophysiological signal from the surface of a body, said system, when in use, to be coupled to a sense electrode arrangement for coupling to the surface of the body such that the sense electrode arrangement and the body define a coupling capacitance (Ce), wherein the sensing system, wherein the sensing system comprises:

a first sensing circuit for sensing (receiving) a first signal at the surface from the sense electrode arrangement and generating a first output based on a first transfer function;
a second sensing circuit for sensing (receiving) a second signal at the surface from the sense electrode arrangement and generating a second output based on a second transfer function, different to the first transfer function; and
a processor adapted to process the first and second outputs thereby to determine the coupling capacitance.

The determination of the coupling capacitance enables the electrode to body (e.g. electrode to skin) coupling to be assessed without injecting an electrical current through the body.

The approach is based on an architecture in which the electrode arrangement (which may be a single electrode or multiple closely spaced electrodes) is coupled to two different sensing circuits with different topologies, each having a distinct transfer function. For example there may be a parallel arrangement of the two sensing circuits.

Each sensing circuit provides an output signal that is based on the same electrophysiological signal (or multiple signals which can be assumed to be the same based on their physical proximity and the temporal closeness of the measurements) and the corresponding transfer functions. The first and second signals sensed by the first and second sensing circuits are thus intended to represent the same physiological parameter of the body and with the same coupling capacitance. The transfer functions are determined by component values of the circuits and are known. The unknowns are the coupling capacitance and the electrophysiological signal (such as voltage, current, or impedance) being measured. The coupling capacitance can be estimated analytically from the two transfer functions with two unknowns. The electrophysiology can be evaluated via sensing electronics for sensing voltages, currents or impedances.

Thus, the two transfer functions have orthogonality in the sense that from the two functions the two independent unknown values can be resolved.

In an embodiment the system comprises the sense electrode arrangement for coupling to the surface of the body such that the sense electrode arrangement and the body define the coupling capacitance (Ce).

The signal being sensed by both sensors is an electrophysiological signal, such as a voltage, current or impedance which is detectable at the skin.

The processor is preferably further adapted to process the first and second outputs thereby to determine the electrophysiological signal being monitored at the surface. Thus, the electrophysiological signal being monitored is determined as well as the coupling capacitance, which is indicative of the coupling quality.

The processor may be adapted to determine the electrophysiological signal being monitored at the surface further taking into account the determined coupling capacitance, thereby to provide body motion compensation. Thus, the coupling capacitance information may be used to compensate for signal variations caused by movement, which movements influence the coupling capacitance.

In one set of examples, the system further comprises a switch arrangement for coupling a selected one of the first and second sensing circuits to the processor and to a shared sense electrode. In this case, there is one electrode (for a pair of sensing circuits), and each sensing circuit is used in a time sharing manner. Thus, in order to create two fully independent transfer functions, the amplifiers are alternately activated via switches that are controlled by the processor.

In another set of examples, the sense electrode arrangement comprises a first electrode connected to the first sensing circuit and a second electrode connected to the second sensing circuit. In this case, separate sensing channels may be used at the same time, if two electrodes are sufficiently close such that the coupling capacitance and the first and second signals may be assumed to be the same.

The first and second sensing circuits (for each sensing electrode) for example each comprise a signal amplifier. In one example, the first sensing circuit comprises a voltage amplifier and the second sensing circuit comprises a charge amplifier. The different types of amplifier, such as buffer amplifiers, provide suitable different transfer functions.

The electrophysiological signal may comprise an ECG signal, such as a fetal ECG signal and a maternal ECG signal. The system may for example be part of a fetal monitoring system.

The electrophysiological signal may comprises an EMG signal. The system may for example be part of a neuromuscular diagnostic system.

The system preferably further comprises an interface adapted to provide electrode contact quality information based on the coupling capacitance. This interface may be a user interface to inform a user that the measurement is not reliable and that the attachment of one or more capacitive electrodes needs to be improved. It may be an interface to a further processing system, to assist in the interpretation of the measured signals.

For example, the sense electrode arrangement may comprise an array of electrodes, and the processor is adapted to determine which electrodes of the array have appropriate coupling to the body based on the respective coupling capacitance.

Thus, poorly coupled electrodes can be excluded from post-processing stages to only include electrodes that are coupled properly to the body and thus provide a reliable measurement.

The invention also provides a method for sensing an electrophysiological signal from a surface of a body, comprising:

sensing (receiving) a first signal at the surface from an sense electrode arrangement, coupled to the surface of the body such that the sense electrode arrangement and the body define a coupling capacitance and generating a first output based on a first transfer function;

sensing (receiving) a second signal at the surface from the sense electrode arrangement and generating a second output based on a second transfer function, different to the first transfer function; and

determining the coupling capacitance from the first and second outputs.

The method may further comprise determining the electrophysiological signal from the first and/or second outputs.

In an embodiment the method further comprises coupling the sense electrode arrangement to the surface of the body such that the sense electrode arrangement and the body define a coupling capacitance (Ce).

The method may further comprise determining the electrophysiological signal at the surface further taking into account the determined coupling capacitance, thereby to provide body motion compensation.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a sensing system for sensing an electrophysiological signal from the body of a subject;
Figure 2 shows a method for sensing an electrophysiological signal from the surface of a body;
Figure 3 shows an example of the switching signals used to control the circuit of Figure 1;
Figure 4 shows a zoomed in region of Figure 3 to show that overlap between the two signals is avoided;
Figure 5 shows a simulation output, where the upper plot is the raw output, while the lower plot is after filtering with a high-pass filter; and
Figure 6 shows an example of the output signals during measurements from a prototype system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensing system and method that uses a sense electrode arrangement for coupling to a surface of a body such that the sense electrode arrangement and the body (and the spacing between them) define a coupling capacitance. The sensing system includes first and second sensing circuits, which have different transfer functions and generate first and second outputs. These outputs are processed to determine the coupling capacitance. In this way, the quality of the electrode coupling can be determined in a simple and passive manner. An electrophysiological signal being monitored is also acquired by one or both of the sensing circuits.

The body is for example the body of a subject and the surface is the skin. This particular use of the invention will be described in the detailed examples below.

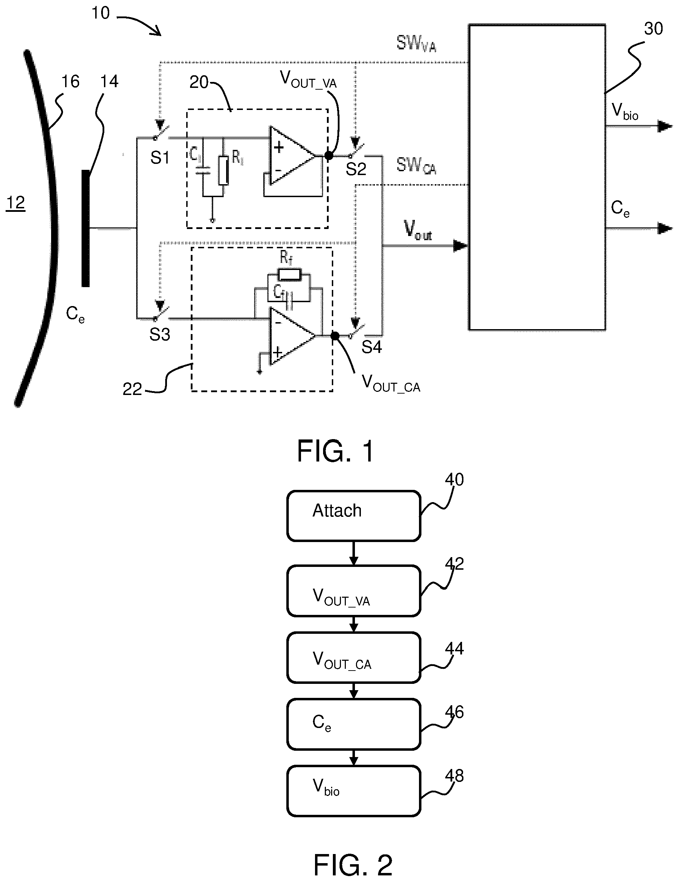

Figure 1 shows a sensing system 10 for sensing an electrophysiological signal from the surface 16 (i.e. skin) of the body 12 of a subject. The sensing system, when is use, is arranged to be coupled to a sense electrode arrangement 14 is provided for capacitive coupling to the surface 16 without galvanic contact, such that the sense electrode arrangement 14, the body 12 and a gap between them define a coupling capacitance Ce. In an embodiment the system may comprise the sense electrode arrangement 14.

There is typically mechanical contact between the sense electrode arrangement and the surface 16, but not galvanic contact. For example, the sense electrode arrangement may be coupled to body tightly but with clothing between. A capacitance is formed by the surface and the electrode arrangement, and the spacing between them. This spacing may be a layer of material which functions as a dielectric or a gap.

A physical capacitor may additionally be present between the surface and the sensing electronics, thus realizing two capacitors in series (the physical capacitor and the spacing). Thus, there may be a galvanic contact to the surface via one plate of a physical capacitor, however, there is no DC signal passing to the sensing electronics and capacitive signal sensing is again employed.

A first sensing circuit 20 is arranged to receive or sense a first electrophysiological signal at the body via the sense electrode arrangement 14 and generating a first output $V_{OUT\_VA}$ based on a first transfer function. A second sensing circuit 22 is arranged to receive or sense a second electrophysiological signal at the body via the same sense electrode arrangement and generating a second output $V_{OUT\_CA}$ based on a second transfer function, different to the first transfer function. These first and second electrophysiological signals are caused by the same physiological process and thus they represent the same physiological parameter to be monitored.

In the example described below, the first and second electrophysiological signals are described as voltages, but the sensing may instead be based on current measurement or impedance measurement.

A processor 30 processes the first and second outputs thereby to determine the coupling capacitance $C_e$. It also determines the electrophysiological signal Vbio being monitored. The electrophysiological signal being monitored may be determined from only one of the sensing circuits (i.e. a preferred one of the sensing circuits, and the less preferred sensing circuit is provided for the purposes of enabling determination of the coupling capacitance). Alternatively, the electrophysiological signal being monitored may be derived from both of the first and second outputs. The determined coupling capacitance may also be taken into account when deriving the physiological parameter of interest from the first and/or second outputs.

In this example, the first sensing circuit 20 is a voltage amplifier. At its input, there is a bias resistor $R_i$ and an input capacitance $C_i$ in parallel. The amplifier is implemented by an operational amplifier with a closed negative feedback path.

The second sensing circuit 22 is a current amplifier. It is implemented by an operational amplifier with a negative feedback path with a parallel bias resistor $R_f$ and capacitance $C_f$.

The capacitances $C_i$ and $C_f$ include parasitic effects of the amplifiers.

Thus, a front-end amplifier architecture is used, with two amplifiers per sensing electrode.

The transfer function for the voltage amplifier 20 is:

$$T_{VA} = \frac{j\omega R_i C_e}{1+j\omega R_i(C_e+C_i)}$$

Hence:

$$T_{VA}(R_i \gg 1) = \frac{C_e}{C_e+C_i} \qquad \text{(Equation 1)}$$

The transfer function for the charge amplifier is:

$$T_{CA} = \frac{j\omega R_f C_e}{1+j\omega R_f C_f}$$

Hence:

$$T_{CA}(R_f \gg 1) = \frac{C_e}{C_f} \qquad \text{(Equation 2)}$$

In the example shown, the two sensing circuits are alternately activated via switches S1 to S4 that are controlled via the processor 30 to create two fully independent transfer functions. The voltage amplifier 20 has a switch S1 at its input and a switch S2 at its output. These switches are controlled via a switching signal $SW_{VA}$. The charge amplifier 20 also has a switch S3 at its input and a switch S4 at its output. These switches are controlled via switching signal $SW_{CA}$.

The processor 30 is thus adapted to switch from one way to acquire the signal using the first sensing circuit to another way using the second sensing circuit. This may be performed both during determination of the coupling capacitance as described above, but also during electrophysiological signal acquisition, in that the sensing may use one or both of the sensing circuits, as mentioned above.

In order for the measurements to be accurate, the switching scheme should be such (in terms of speed and duration) that the properties of the electrophysiological signal being measured can be assumed as constant.

Each amplifier provides an output signal ($V_{OUT\_VA}$ and $V_{OUT\_CA}$) that is based on the same electrophysiological signal (Vbio) and the corresponding transfer functions ($T_{VA}$ and $T_{CA}$).

The output signal of the voltage amplifier is thus:

$$V_{OUT\_VA} = V_{bio} \cdot \frac{C_e}{C_e + C_i} \qquad \text{(Equation 3)}$$

The output signal of the charge amplifier is:

$$V_{OUT\_CA} = V_{bio} \cdot \frac{C_e}{C_f} \qquad \text{(Equation 4)}$$

In Equation 3 and 4, $C_i$ and $C_f$ are circuit values which are either known by design or can be estimated by a calibration procedure. Such a calibration can be an integral part of the electronics circuit and can be performed whenever required, e.g. based on temperature variations as this might cause drifts in $C_i$ and $C_f$.

In the calibration procedure, $C_i$ and $C_f$ can be estimated with Equation 3 and 4 respectively. In this case $V_{bio}$ may be set temporarily to be a known AC signal generated by the circuit itself. In order to have a correct estimation, the frequency of the generated signal should be within the band of the electrophysiological signal of interest, e.g. 100Hz for an ECG signal.

For $C_e$, a real (e.g. on-chip, off-the-shelf) capacitor may be used for such a calibration (with typical values in the range: 1-100pF).

By means of substitution, Equations 3 and 4 can be combined to derive equation 5 in order to estimate the electrode-skin capacitive coupling:

$$\hat{C}_e = \frac{V_{OUT\_CA}}{V_{OUT\_VA}} C_f - C_i \qquad \text{(Equation 5)}$$

It is beneficial to switch between the sensing circuits with high frequency in order to improve the accuracy. Higher frequency switching can detect faster changes in the skin-electrode capacitance $C_e$. Further, it also assures that the electrophysiological signal change that the two amplifies measure is minimal. However, there is also an upper limit to the switching frequency in that if the frequency is too high, the measurement results will be degraded due to the finite speed of the sensing circuits, switches, and communications interface. Thus, there is a range of suitable switching frequencies as will be apparent to those skilled in the art.

The estimated value of the coupling capacitance can be used to control the switching of the amplifiers and decide on the usability of the acquired signal.

Figure 2 shows a corresponding method for sensing a signal from the surface of a body. The method comprises, in step 40, coupling a sense electrode arrangement to a surface of the body such that the sense electrode arrangement and the body (and the spacing between them) define a coupling capacitance (Ce).

In step 42, a first signal at the body is sensed via the sense electrode arrangement and a first output is generated based on a first transfer function (the voltage amplifier).

In step 44, a second signal at the body via the sense electrode arrangement and a second output is generated based on a second transfer function (the charge amplifier).

In step 46, the coupling capacitance $C_e$ is determined from the first and second outputs.

In step 48, the electrophysiological signal ($V_{bio}$) is determined from the first and/or second outputs.

In order to prevent overload or short-circuits, the timing of the switching signals is selected such that there is no overlap during the 'ON' period of the switches.

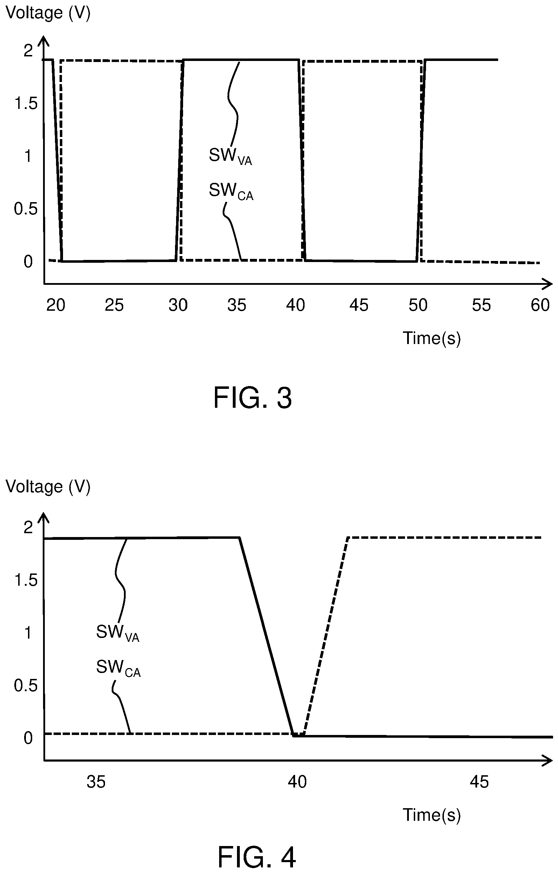

Figure 3 shows an example of the switching signals $SW_{VA}$ and $SW_{CA}$. Figure 4 shows a zoomed in region to show that overlap between the two signals is avoided.

The proof-of-principle of the approach of invention has been demonstrated first by simulations and second by laboratory measurements of a built prototype, and the results are presented below.

Simulation

The circuit of Figure 1 has been simulated. As an example, values were chosen of $C_e$ = 20pF and $C_e$ = 100pF

as two calibration points, Simulations were performed and the unknown parameters $C_i$ and $C_f$ were determined according to Equations 3 and 4, which turn out to be:

$$C_i = 1.1pF$$

$$C_f = 5.5pF$$

In order to estimate an unknown electrode capacitor $C_e$, the combined amplifier is switched as explained above.

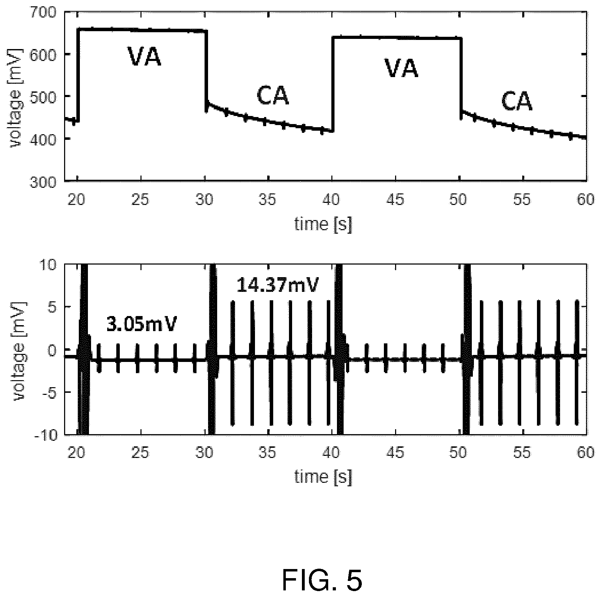

Figure 5 shows the simulation output, where the upper plot is the raw output, while the lower plot is after filtering with a high-pass filter (cut-off at 3Hz) to remove the DC component.

The filtered plot shows a voltage amplifier output amplitude of 3.05mV. However, the designed voltage amplifier has a built-in gain of 2, so that after correcting for this gain the real voltage amplifier output amplitude is:

$$V_{OUT\_VA} = 1.525mV$$

The plot furthermore shows a current amplifier output amplitude:

$$V_{OUT\_CA} = 14.37mV$$

By means of Equation 5, the electrode-skin capacitive coupling can be estimated as follows:

$$\hat{C}_e = \frac{V_{OUT\_CA}}{V_{OUT\_VA}} C_f - C_i = \frac{14.37mV}{1.525mV} * 5.5pF - 1.1pF = 50.7pF$$

In this simulation the true value for $C_e$ was 50pF. Thus, the deviation from the estimated value is 0.7pF, which is mostly due to rounding errors.

The table below shows the results for other (true vs. predicted) values of $C_e$.

| $C_e$ | |
|---|---|
| **True value [pF]** | **Prediction value [pF]** |
| 1 | 1.3 |
| 5 | 5.3 |
| 10 | 10.3 |
| 50 | 50.7 |
| 74 | 73.2 |

Lab measurement

A prototype of the front-end amplifier architecture was designed and fabricated as an ASIC, and integrated into a capacitive sensing system. Various capacitors (with different values) were soldered to the amplifier's input and connected to an ECG signal generator.

The true values of these capacitors had been measured with a capacitor-meter with 0.1 pF accuracy.

First, known capacitors $C_e = 1.6pF$ and $C_e = 104.2pF$ were taken as calibration points, which led to $C_i = 7.26pF$ and $C_f = 7.71pF$. Next, a series of 'unknown' coupling capacitors were tested.

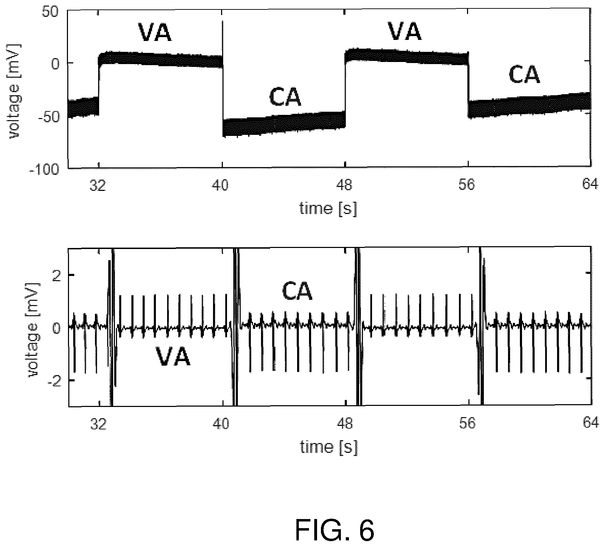

Figure 6 shows an example of the output signals during measurements. Again the raw signals are shown at the top and filtered signals are shown at the bottom.

The table below shows that the estimated values are again close to the real values of the coupling capacitor.

| Ce | |
|---|---|
| Value [pF] | Prediction [pF] |
| 0.6 | 0.7 |
| 2.4 | 2.3 |
| 4.9 | 4.8 |
| 10.2 | 10.2 |
| 22.1 | 22.0 |
| 47.0 | 46.8 |

The example above is based on a voltage amplifier and a current amplifier. However, any two sensing circuits may be used as long as they provide two independent equations from which the electrophysiological signal and the coupling capacitance may be separately resolved.

The sensing circuits may be other kinds of amplifiers (or even non-amplifier circuits) that have a different transfer function.

The example above is based on a single electrode. However, there may be an electrode arrangement with two (or more) separate electrodes, with two separate (and different) amplifiers. One electrode may be connected to a first type of sensing circuit and the other electrode may be connected to a second type of sensing circuit. As long as the electrodes are spaced closely enough, the electrophysiological signal $V_{bio}$ correlation will be sufficiently good to enable estimation of $C_e$ (which can also be assumed to be the same for the two electrodes). The advantage is that measurement can be made using both transfer functions simultaneously, without needing a switch arrangement and corresponding control.

If the coupling impedance is complex (i.e. not just a capacitance, but a combination of resistance and capacitance), a system of three measurement systems with three different transfer functions may be used to resolve the real and imaginary impedances as well as the electrophysiological signal.

The invention is relevant for all applications wherein electrophysiological signals (e.g., ECG, EMG) are measured. Example applications are contactless fetal monitoring and neonatal monitoring.

The invention is also applicable to other contactless sensing applications not necessarily related to electrophysiological signals. Thus, the invention may be applied more generally to contactless capacitive sensing of a signal at any surface.

Two particularly relevant application examples are described below that could benefit significantly from capacitive electrophysiological measurements.

A first example is neonatal monitoring in the neonatal intensive care unit. Although new monitoring technologies in neonatal care have improved the survival rates of preterm neonates, the damage caused by the invasive aspect of such techniques could affect the neonates' development. The thin skin of preterm babies is easily damaged by adhesive electrodes. Capacitive electrodes present an attractive option for pervasively monitoring neonatal ECG, and can be embedded in a support system or even a garment worn by the neonates. This could improve comfort aiding better recovery as well as avoiding the scars caused by adhesive electrodes.

For example, reference is made to Atallah, L. et al. Unobtrusive ECG monitoring in the NICU using a capacitive sensing array. Physiol. Meas. 35, 895-913 (2014).

A second example is pregnancy (fetal and maternal) monitoring. In conventional non-invasive fetal monitoring systems a Doppler ultrasound device is used to measure fetal cardiac activity. Contractions are monitored using a pressure sensor that is usually based on a strain gauge. These systems are quite bulky, limit the mobility of pregnant women and require a trained professional for correct application and operation. This means that these systems can only be used in clinical settings such as in hospitals. Over the years many studies have been published on the positive effect of ambulation during pregnancy, e.g. that walking and upright positions in the first stage of labor reduce the length of labor. Cable-less fetal monitoring systems exist which solve the issue of mothers-to-be being 'cabled' at the bedside. Furthermore, wearable fetal monitoring solutions in the form of a patch have also been developed based on electrophysiological measurements. The fetal and maternal heart rate is derived from an ECG measurement using wet electrodes attached to the abdomen of a pregnant woman. The same electrodes are used to derive uterine contractions from an EMG measurement. These new wearable monitoring solutions improve the patient's mobility and comfort even further since the measurement patches are very lightweight, easy to apply and do not require repositioning of the electrodes during operation. Unfortunately, these electrophysiological patches are still not optimal for home-monitoring. This is mainly due to challenges relating to ease-of-use (e.g. skin scrubbing with sandpaper, correct positioning of the patch), comfort (e.g. adhesive gel electrodes irritate the skin), and reliability of the measurement (in contrast to a hospital setting, the home is an uncontrolled environment, i.e. lack of contextual information).

Also in fetal monitoring applications, capacitive electrophysiological sensors have the potential to be a comfortable alternative, as they do not require skin preparation or direct skin contact. They can be integrated in wearable patches or easily embedded in pregnancy belts/pant to allow measurements through thin electrically insulating layers (e.g. textiles). Ultimately this will enable solutions that can monitor at-risk pregnant women in a home environment, close to their families and furthermore offload hospitals resulting in a decrease in healthcare costs. The feasibility of extracting fetal and maternal ECG (and heart rate) using capacitive electrophysiological sensors has been demonstrated for example in E. Rendon-Morales, R. J. Prance, and R. Aviles-Espinosa. Non-invasive recordings of fetal electrocardiogram during pregnancy using electric potential sensors. American Institute of Physics. 12 October 2018.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

**Claims**

1. A sensing system (10) for sensing an electrophysiological signal from the surface of a body, said system, when in use, to be coupled to a sense electrode arrangement (14) for coupling to the surface (16) of the body (12) such that the sense electrode arrangement and the body define a coupling capacitance (Ce), wherein the sensing system comprises:

   a first sensing circuit (20) arranged to sense a first signal at the surface via the sense electrode arrangement and generate a first output ($V_{OUT\_VA}$) based on a first transfer function;
   a second sensing circuit (22) arranged to sense a second signal at the surface via the sense electrode arrangement and generate a second output ($V_{OUT\_CA}$) based on a second transfer function, different to the first transfer function; and
   a processor (30) adapted to process the first and second outputs thereby to determine the coupling capacitance (Ce).

2. The sensing system as claimed in claim 1, wherein the processor (30) is further adapted to process the first and/or second outputs ($V_{OUT\_VA}$, $V_{OUT\_CA}$) thereby to determine the electrophysiological signal ($V_{bio}$) at the surface.

3. The sensing system as claimed in claim 2, wherein the processor is adapted to determine the electrophysiological signal ($V_{bio}$) at the surface further taking into account the determined coupling capacitance, thereby to provide body motion compensation.

4. The sensing system as claimed in any one of claims 1 to 3, further comprising a switch arrangement (S1-S4) for coupling a selected one of the first and second sensing circuits to the processor and to a shared sense electrode (14).

5. The sensing system as claimed in any one of claims 1 to 3, further comprising the sense electrode arrangement wherein the sense electrode arrangement comprises a first electrode connected to the first sensing circuit (20) and a second electrode connected to the second sensing circuit (22).

6. The sensing system as claimed in claim 5, wherein the sense electrode arrangement comprises a first electrode connected to the first sensing circuit (20) and a second electrode connected to the second sensing circuit (22).

7. The sensing system as claimed in any one of claims 1 to 6, wherein the first and second sensing circuits (20, 22) each comprise an amplifier circuit.

8. The sensing system as claimed in claim 7, wherein the first sensing circuit (20) comprises a voltage amplifier and the second sensing circuit (22) comprises a charge amplifier.

9. The sensing system as claimed in any one of claims 1 to 8, wherein the signal comprises an ECG signal.

10. The sensing system as claimed in claim 9, wherein the signal comprises a fetal ECG signal and a maternal ECG signal.

11. The sensing system as claimed in any one of claims 1 to 8, wherein the signal comprises an EMG signal.

12. The sensing system as claimed in any one of claims 1 to 11, further comprising an interface adapted to provide electrode contact quality information based on the coupling capacitance.

13. The sensing system as claimed in any one of claims 5 to 12, wherein the sense electrode arrangement comprises an array of electrodes, and wherein the processor (30) is adapted to determine which electrodes of the array have appropriate coupling to the body based on the respective coupling capacitance.

14. A method for sensing an electrophysiological signal from the surface of a body, comprising:

(42) sensing a first signal at the surface via a sense electrode arrangement, coupled to the surface of the body such that the sense electrode arrangement and the body define a coupling capacitance ($C_e$); and generating a first output ($V_{OUT\_VA}$) based on a first transfer function;
(42) sensing a second signal at the surface via the sense electrode arrangement and generating a second output ($V_{OUT\_CA}$) based on a second transfer function, different to the first transfer function; and determining the coupling capacitance (Ce) from the first and second outputs.

15. The method as claimed in claim 14 further comprising coupling the sense electrode arrangement to the surface of the body such that the sense electrode arrangement and the body define a coupling capacitance (Ce).

16. The sensing method as claimed in claims 14 to 15, comprising determining the electrophysiological signal (Vbio) at the surface from the first and/or second outputs.

17. The method as claimed in claim 16, comprising determining the electrophysiological signal at the surface further taking into account the determined coupling capacitance, thereby to provide body motion compensation.

10

16  14   20        $V_{OUT\_VA}$

SW$_{VA}$

30

12

$C_i$  $R_i$

S1

S2   SW$_{CA}$

$V_{bio}$

$V_{out}$

$C_e$

$R_f$

$C_f$

S3

S4

22

$V_{OUT\_CA}$

$C_e$

## FIG. 1

Attach                    40

$V_{OUT\_VA}$              42

$V_{OUT\_CA}$             44

$C_e$                     46

$V_{bio}$                 48

## FIG. 2

Voltage (V)

FIG. 3

Voltage (V)

FIG. 4

FIG. 5

FIG. 6

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 783 725 A1 (YANG CHANGMING [CN]) 1 October 2014 (2014-10-01) * abstract * * paragraphs [0001], [0010] - [0011], [0014], [0136] - [0138], [0154] - [0155], [0159], [0162] * * figures 19,23,29 * * claim 69 * | 1-9, 11-17 | INV. A61B5/0428 A61B5/00 |
| X | XU LIN ET AL: "Motion-Artifact Reduction in Capacitive Heart-Rate Measurements by Adaptive Filtering", IEEE TRANSACTIONS ON INSTRUMENTATION AND MEASUREMENT, IEEE SERVICE CENTER, PISCATAWAY, NJ, US, vol. 68, no. 10, 1 October 2019 (2019-10-01), pages 4085-4093, XP011745599, ISSN: 0018-9456, DOI: 10.1109/TIM.2018.2884041 [retrieved on 2019-09-12] | 1,9, 14-16 | |
| A | * abstract * * page 4086, left-hand column, paragraph 2 * * eq.1; page 4086 * * eq.2; page 4087 * * page 4087, right-hand column * * sect.II.C.2-3; page 4088 * * figure 4 * | 2-8, 10-13,17 | TECHNICAL FIELDS SEARCHED (IPC) A61B |

-/--

The present search report has been drawn up for all claims

| Place of search | Date of completion of the search | Examiner |
|---|---|---|
| The Hague | 16 July 2020 | Delval, Christophe |

EPO FORM 1503 03.82 (P04C01)

Europäisches
Patentamt

European
Patent Office

Office européen
des brevets

# EUROPEAN SEARCH REPORT

Application Number

EP 20 15 4351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XU LIN ET AL: "Adaptive motion-artifact reduction in capacitive ECG measurements by using the power-line interference", 2018 IEEE INTERNATIONAL SYMPOSIUM ON MEDICAL MEASUREMENTS AND APPLICATIONS (MEMEA), IEEE, 11 June 2018 (2018-06-11), pages 1-5, XP033387793, DOI: 10.1109/MEMEA.2018.8438751 [retrieved on 2018-08-16] | 1,14 | |
| A | * abstract * <br> * sect.II, IV * | 2-13, 15-17 | |
| X | EP 2 453 792 A1 (KONINKL PHILIPS ELECTRONICS NV [NL]) 23 May 2012 (2012-05-23) | 1,9,10, 14-16 | |
| A | * abstract * <br> * paragraphs [0096] - [0098], [0165] * <br> * claims 1,13,14 * | 2-8, 11-13,17 | |
| X | EP 2 429 390 A1 (NOX MEDICAL [IS]) 21 March 2012 (2012-03-21) * abstract * * paragraph [0009] * * claims 11-13 * | 1,9, 14-16 | TECHNICAL FIELDS SEARCHED (IPC) |
| X | WO 2015/061282 A1 (UNIV CALIFORNIA [US]) 30 April 2015 (2015-04-30) * abstract * * paragraph [0019] * * claims 1-6 * | 1,9, 14-16 | |
| X | WO 2013/072839 A2 (KONINKL PHILIPS ELECTRONICS NV [NL]) 23 May 2013 (2013-05-23) * abstract * * claims 1-4 * | 1,9, 14-16 | |

-/--

The present search report has been drawn up for all claims

| Place of search | Date of completion of the search | Examiner |
|---|---|---|
| The Hague | 16 July 2020 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/364184 A1 (WEINERTH JOHN [US] ET AL) 21 December 2017 (2017-12-21)<br>* abstract *<br>* figure 11 *<br>----- | 1,9, 14-16 | |
| X | US 2018/206790 A1 (OEHLER MARTIN [DE] ET AL) 26 July 2018 (2018-07-26)<br>* abstract *<br>* claims 1,8 *<br>----- | 1,9, 14-16 | |
| X | CN 107 788 968 A (UNIV CENTRAL SOUTH) 13 March 2018 (2018-03-13)<br>* abstract *<br>* paragraphs [0005], [0007], [0031] *<br>* claims 1,8 *<br>----- | 1,9, 14-16 | |
| X | WO 02/13690 A1 (STANALAND THOMAS G [US]; CHEVALIER GAETAN [US]) 21 February 2002 (2002-02-21)<br>* abstract *<br>* page 16, line 11 - page 17, line 14 *<br>* page 18, line 25 - line 31 *<br>* claim 1 *<br>----- | 1,9, 14-16 | TECHNICAL FIELDS SEARCHED (IPC) |
| A | YONG GYU LIM ET AL: "Capacitive Measurement of ECG for Ubiquitous Healthcare", ANNALS OF BIOMEDICAL ENGINEERING, vol. 42, no. 11, 23 July 2014 (2014-07-23) , pages 2218-2227, XP055715139, New York ISSN: 0090-6964, DOI: 10.1007/s10439-014-1069-6<br>* abstract *<br>* figures 1,8 *<br>* the whole document *<br>----- | 1-17 | |

-/--

The present search report has been drawn up for all claims

| Place of search | Date of completion of the search | Examiner |
|---|---|---|
| The Hague | 16 July 2020 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 15 4351

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YU MIKE CHI ET AL: "Dry-Contact and Noncontact Biopotential Electrodes: Methodological Review", IEEE REVIEWS IN BIOMEDICAL ENGINEERING, vol. 3, 1 January 2010 (2010-01-01), pages 106-119, XP055715153, USA ISSN: 1937-3333, DOI: 10.1109/RBME.2010.2084078 * abstract * * figures 2,3 * * the whole document * | 1-17 | |
| A | ENRIQUE SPINELLI ET AL: "A capacitive electrode with fast recovery feature", PHYSIOLOGICAL MEASUREMENT., vol. 33, no. 8, 20 July 2012 (2012-07-20), pages 1277-1288, XP055715346, GB ISSN: 0967-3334, DOI: 10.1088/0967-3334/33/8/1277 * abstract * * figures 1-7 * * the whole document * | 1-17 | |

TECHNICAL FIELDS
SEARCHED      (IPC)

The present search report has been drawn up for all claims

| Place of search | Date of completion of the search | Examiner |
|---|---|---|
| The Hague | 16 July 2020 | Delval, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

page 4 of 4

## ANNEX TO THE EUROPEAN SEARCH REPORT
## ON EUROPEAN PATENT APPLICATION NO.

EP 20 15 4351

This annex lists the patent family members relating to the patent documents cited in the above-mentioned European search report.
The members are as contained in the European Patent Office EDP file on
The European Patent Office is in no way liable for these particulars which are merely given for the purpose of information.

16-07-2020

| Patent document cited in search report | | Publication date | Patent family member(s) | | Publication date |
|---|---|---|---|---|---|
| EP 2783725 | A1 | 01-10-2014 | EP | 2783725 A1 | 01-10-2014 |
| | | | JP | 6169592 B2 | 26-07-2017 |
| | | | JP | 2015504338 A | 12-02-2015 |
| | | | JP | 2017140411 A | 17-08-2017 |
| | | | JP | 2017213391 A | 07-12-2017 |
| | | | KR | 20140098809 A | 08-08-2014 |
| | | | KR | 20160108587 A | 19-09-2016 |
| | | | KR | 20160108588 A | 19-09-2016 |
| | | | US | 2014343392 A1 | 20-11-2014 |
| | | | WO | 2013075270 A1 | 30-05-2013 |
| | | | WO | 2013075388 A1 | 30-05-2013 |
| EP 2453792 | A1 | 23-05-2012 | CN | 102469949 A | 23-05-2012 |
| | | | EP | 2453792 A1 | 23-05-2012 |
| | | | JP | 5602227 B2 | 08-10-2014 |
| | | | JP | 2012532731 A | 20-12-2012 |
| | | | US | 2012116198 A1 | 10-05-2012 |
| | | | WO | 2011007292 A1 | 20-01-2011 |
| EP 2429390 | A1 | 21-03-2012 | EP | 2429390 A1 | 21-03-2012 |
| | | | US | 2012101357 A1 | 26-04-2012 |
| | | | US | 2016073921 A1 | 17-03-2016 |
| | | | WO | 2010131267 A1 | 18-11-2010 |
| WO 2015061282 | A1 | 30-04-2015 | US | 2016256111 A1 | 08-09-2016 |
| | | | WO | 2015061282 A1 | 30-04-2015 |
| WO 2013072839 | A2 | 23-05-2013 | NONE | | |
| US 2017364184 | A1 | 21-12-2017 | NONE | | |
| US 2018206790 | A1 | 26-07-2018 | DE | 102015111658 A1 | 19-01-2017 |
| | | | EP | 3324827 A1 | 30-05-2018 |
| | | | JP | 2018524116 A | 30-08-2018 |
| | | | KR | 20180030652 A | 23-03-2018 |
| | | | US | 2018206790 A1 | 26-07-2018 |
| | | | WO | 2017012987 A1 | 26-01-2017 |
| CN 107788968 | A | 13-03-2018 | NONE | | |
| WO 0213690 | A1 | 21-02-2002 | AU | 8642201 A | 25-02-2002 |
| | | | US | 2002038092 A1 | 28-03-2002 |
| | | | WO | 0213690 A1 | 21-02-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ATALLAH, L. et al.** Unobtrusive ECG monitoring in the NICU using a capacitive sensing array. *Physiol. Meas.,* 2014, vol. 35, 895-913

- **E. RENDON-MORALES ; R. J. PRANCE ; R. AVILES-ESPINOSA.** Non-invasive recordings of fetal electrocardiogram during pregnancy using electric potential sensors. American Institute of Physics, 12 October 2018